(19) Europäisches Patentamt
European Patent Office
Office européen des brevets

(11) EP 2 652 042 B1

(12) # FASCICULE DE BREVET EUROPEEN

(45) Date de publication et mention de la délivrance du brevet:
**09.11.2022 Bulletin 2022/45**

(21) Numéro de dépôt: **11808890.5**

(22) Date de dépôt: **12.12.2011**

(51) Classification Internationale des Brevets (IPC):
***C08L 83/04*** *(2006.01)* ***A61K 6/90*** *(2020.01)*
*C08G 77/12* *(2006.01)* *C08G 77/20* *(2006.01)*

(52) Classification Coopérative des Brevets (CPC):
(C-Sets disponibles)
**A61K 6/90; C08L 83/04;** C08G 77/12; C08G 77/20

(Cont.)

(86) Numéro de dépôt international:
**PCT/FR2011/000644**

(87) Numéro de publication internationale:
**WO 2012/080594 (21.06.2012 Gazette 2012/25)**

(54) **MATERIAU ELASTOMERE SILICONE UTILISABLE NOTAMMENT POUR LA PRISE D'EMPREINTES DENTAIRES**

SILIKONELASTOMERSTOFF ZUR VERWENDUNG INSBESONDERE BEI DER HERSTELLUNG VON ZAHNABDRÜCKEN

SILICONE ELASTOMER MATERIAL SUITABLE FOR USE IN PARTICULAR FOR MAKING DENTAL IMPRESSIONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.12.2010 FR 1004844**

(43) Date de publication de la demande:
**23.10.2013 Bulletin 2013/43**

(73) Titulaire: **ELKEM SILICONES France SAS**
**69003 Lyon (FR)**

(72) Inventeur: **DEL TORTO, Marco**
**20151 Milano (IT)**

(74) Mandataire: **Mekki, Boualem et al**
**Elkem Silicones France SAS**
**9, rue Spécia**
**69190 Saint-Fons (FR)**

(56) Documents cités:
**WO-A1-93/17654** **DE-A1- 19 801 657**
**US-A1- 2006 089 427** **US-B1- 6 376 569**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 652 042 B1

(52) Classification Coopérative des Brevets (CPC):
(Cont.)

C-Sets
**A61K 6/90, C08L 83/04;**
**C08L 83/04, C08L 3/00;**
**C08L 83/04, C08L 83/04**

## Description

Le domaine de la présente invention est celui des compositions d'organopolysiloxanes susceptibles de réticuler et/ou durcir par des réactions de polyaddition pour former des élastomères ou des matériaux en silicone utiles notamment pour la prise d'empreinte dentaire dans le cadre de la réalisation de prothèses. Ces élastomères ou ces matériaux en silicone sont utilisés pour prendre une empreinte intra-orale pour une prothèse dentaire telle qu'une couronne, un « inlay » ou un appareil dentaire.

Par l'expression "prise d'empreintes", on entend définir dans le présent mémoire: non seulement les opérations de prise d'empreintes de n'importe quel objet et de n'importe quelle forme pour réaliser un modèle en particulier en plâtre ; mais encore les opérations de reproductions ou de duplications de modèles en particulier en plâtre. Par l'expression "prise d'empreintes dentaires", on entend définir dans le présent mémoire : non seulement les opérations où l'on procède à la prise d'empreintes dentaires en bouche pour obtenir des reproductions exactes de mâchoires ou de parties de mâchoires portant ou non, et en totalité ou en partie, des dents et pour former des modèles en plâtre ; mais encore les opérations de duplications où l'on procède à la reproduction de modèles de mâchoires ou de parties de mâchoires en plâtre dans un laboratoire de prothèse dentaire. Les applications visées englobent encore la fabrication de pièces moulées, autres que des masses de duplication dans les applications dentaires.

Dans le domaine dentaire, on utilise de plus en plus des élastomères ou matériaux en silicone obtenus par réticulation et/ou durcissement à température ambiante, comme moule utile pour la préparation de prothèses dentaires. Le précurseur de ce matériau est une composition ayant la consistance d'une pâte dans laquelle on introduit un agent de durcissement avant l'emploi. La masse résultante perd alors sa consistance pâteuse pour prendre une consistance « caoutchouteuse » et élastique au cours du durcissement à la température du corps du patient. Du fait de son élasticité, il est alors facile d'enlever l'élastomère en silicone durci de la bouche et on peut ensuite l'utiliser pour y couler un modèle de travail, par exemple en plâtre, à partir duquel on réalise la prothèse dentaire définitive. Ainsi l'utilisation des élastomères silicone est largement répandue dans le domaine dentaire. Ceci est aussi dû en partie au fait que les élastomères ou matériaux en silicone offrent, d'une part une grande diversité de caractéristiques chimiques, mécaniques et physiques, et, d'autre part, un caractère non toxique, non irritant et non allergisant. En outre, les matériaux silicones constituent de piètres substrats de culture pour les micro-organismes, ce qui leur confère des aptitudes remarquables au regard de l'hygiène.

Les compositions de polyorganosiloxanes réticulant par polyaddition sont en général préférés dans le domaine de l'impression dentaire car elles permettent d'obtenir des élastomères ou matériaux silicone de meilleure qualité tant sur le plan des propriétés mécaniques que sur la précision des formes reproduites. Ces compositions sont commercialisées en deux parties distinctes devant être mélangées au moment de l'emploi et sont communément appelées compositions bi-composantes. Dans de telles compositions formulées en deux parties distinctes, une des parties comprend au moins un polyorganosiloxane présentant, par molécule, au moins deux groupes alcényle liés chacun à un atome de silicium et un catalyseur qui est en général un composé d'un métal du groupe du platine alors que dans l'autre partie on trouve au moins polyorganosiloxane présentant, par molécule, au moins deux atomes d'hydrogène liés à un atome de silicium. La réaction de polymérisation, de réticulation ou de durcissement de telles compositions démarre lorsque l'on mélange les deux parties c'est-à-dire le catalyseur à base de platine, le polyorganosiloxane présentant, par molécule, au moins deux atomes d'hydrogène liés à un atome de silicium et le polyorganosiloxane présentant, par molécule, au moins deux groupes alcényle. La vitesse de réticulation varie en fonction des quantités de catalyseur. On peut aussi ajuster la réactivité de la composition en ajoutant des inhibiteurs ou des ralentisseurs de la réaction de polyaddition. Ces composés sont bien connus et agissent de manière concurrentielle avec le catalyseur ralentissant ainsi la réaction de réticulation.

Après mélange des deux parties, la composition résultante commence à durcir et la viscosité du mélange augmente, une gélification (« prise en gel ») démarre jusqu'à la transformation de manière irréversible en un polymère réticulé ou « élastomère ». Lorsque l'on atteint ce point de prise en gel, la composition s'écoule difficilement et à un certain moment la composition ne peut plus être mise en forme par l'utilisateur. On définit ainsi une « période de travail » ou « temps de travail » dans laquelle la composition reste manipulable pour une application telle qu'une prise d'empreinte dentaire. Une fois la réaction terminée (ou pratiquement terminée) on dit que le matériau ou l'élastomère a durcit. Ce temps de durcissement est aussi un paramètre important pour un matériau d'empreinte en silicone car il doit rester en contact avec la surface à répliquer jusqu'à ce qu'il soit complètement durci. Dans le domaine des empreintes dentaires le temps de travail requis est souvent de l'ordre de quelques minutes.

Ainsi pour des applications qui requièrent la reproduction de détails, tels que la prise d'empreinte dentaire, les paramètres de temps de durcissement et de temps de travail doivent être contrôlés avec soin. Cependant, un des facteurs qui affecte à la fois le temps de travail et le temps de durcissement est l'activité du catalyseur. Il est connu que les catalyseurs à base de platine sont sensibles à une longue durée de stockage (un an ou plus) ce qui peut entrainer une dégradation des cinétiques de réticulation. Cela se traduit par une augmentation du temps de durcissement. Ce temps de durcissement devient de plus en plus long, voire inacceptable. En effet, une variation incontrôlée du temps

de durcissement met en péril l'exactitude d'une empreinte si l'utilisateur retire le matériau en silicone du modèle à copier avant qu'il ne soit complètement polymérisé. Pire, on observe aussi des phénomènes de noircissement de la partie contenant le catalyseur au platine lors d'un stockage prolongé la rendant inutilisable.

Il est à noter que ce problème récurrent a fait l'objet de diverses approches. Par exemple, dans les brevets US n° 5,047,444, 5,118,559 et 5,182,316 le niveau de polymérisation est contrôlé par détection d'un fluorophore ultraviolet qui est généré pendant la polymérisation. Cependant, cette approche nécessite l'installation d'appareillages supplémentaires tels qu'une source de rayonnement UV et un détecteur de fluorescence UV.

La demande de brevet DE-19801657-A1 décrit la préparation d'un dérivé obtenu en faisant réagir en milieu aqueux, pendant 3 heures et de préférence à 60°C, une sélection de composés (tels que l'amidon, l'amylose, la cellulose) avec un dérivé du platine. Après filtration et évaporation sous pression réduite, il a été obtenu un produit solide, de couleur jaune et ayant une teneur en Pt comprise entre 1 et 1,5% (voir Exemples 1 et 2) identifié comme le composant (D). Les produits de départ (par exemple l'amidon) ont donc été éliminés par des méthodes de purification standard et ne sont donc plus présent dans l'additif préparé. De plus, Les compositions décrites sont des compositions monocomposantes.

Le brevet US 6,376,569 B1 décrit des compositions polysiloxanes notamment utilisée pour la prise d'empreintes dentaires. Lesdites compositions polysiloxanes sont obtenues par réaction d'hydrosilylation activée par une irradiation ultraviolette ou visible, à l'aide d'une catalyseur au platine spécifique.

La demande internationale WO 93/17654 A1 décrit des matériaux pour impression dentaire ayant une résistance à la déchirure améliorée grâce à l'ajout d'un polymère siloxane mono- et quadri-fonctionnel.

Dans un autre domaine technique, la demande de brevet US 2006/089427 A1 décrit un agent de traitement du papier.

Dans un tel contexte technique, l'un des objectifs essentiels de la présente invention est de palier aux problèmes rencontrés après un long stockage (1an ou plus) des compositions bi-composante de polyaddition et catalysée par un composé d'un métal du groupe du platine. En effet, ces compositions présentent alors un ralentissement de la cinétique de la réaction de polyaddition ce qui se traduit par un allongement du temps de travail et par une dégradation des propriétés physiques de l'élastomère.

Un deuxième objectif de l'invention est de supprimer les phénomènes de noircissement observés après un long stockage (1an ou plus).de la partie comprenant un catalyseur, qui est un composé d'un métal du groupe du platine, d'une composition bi-composante réticulant par des réactions de polyaddition.

Pour atteindre cet objectif, parmi d'autres, les inventeurs ont eu le mérite de mettre à jour de manière tout à fait surprenante et inattendue, que l'addition dans des quantités suffisantes d'un additif spécifique, appelé agent stabilisant dans le présent mémoire, permet de palier aux problèmes exposés ci-dessus.

Ainsi, la présente invention, prise dans son premier objet, concerne une composition **X** réticulable et/ou durcissable par des réactions de polyaddition se présentant sous la forme d'un système bicomposant **S** comprenant au moins deux parties distinctes **A** et **B** destinées à être mélangées pour former ladite composition **X** dans lequel :

- la partie **A** comprend :

  (a) de 30 à 49,5 % en poids par rapport au poids total de la partie **A** d'au moins un agent stabilisant **D** choisi parmi le groupe des amidons,
  (b) au moins un polyorganosiloxane **V** présentant, par molécule, au moins deux groupes alcényle, de préférence vinyle, liés chacun à un atome de silicium,
  (c) au moins un catalyseur **C** qui est un composé d'un métal du groupe du platine, et

- la partie **B** comprend au moins un polyorganosiloxane **H** présentant, par molécule, au moins deux atomes d'hydrogène liés à un atome de silicium identique ou différent.

La composition selon l'invention présente l'avantage d'être stable au stockage et ceci même dans des conditions extrêmes de température et d'humidité. Par « stable au stockage », on comprend que :

- la cinétique de la réaction de polyaddition catalysé par le platiner n'est pas affecté par un long stockage, et
- la suppression du phénomène de noircissement de la partie contenant le catalyseur qui est un composé d'un métal du groupe du platine.

L'amidon est une macromolécule glucidique présente chez de nombreux végétaux, notamment dans les organes de réserve comme le tubercule de pomme de terre et dans les semences (blé, maïs, riz, etc.) où il constitue une forme de stockage du glucose. Dans la molécule d'amidon, des unités glucose reliées par des liaisons osidiques alpha 1-4 forment des chaînes hélicoïdales d'amylose, sur lesquelles de courtes chaînes de même constitution se branchent

par des liaisons osidiques alpha 1-6. Dans l'amidon, ces ramifications sont présentes environ tous les trente résidus glucose. En présence d'iode, l'amidon se colore en bleu-violet.

Le terme « amidon » comprend aussi les amidons dits « modifiés » tels que :

- un amidon traité aux acides ;
- un amidon traité aux bases ;
- un amidon blanchi ;
- une fécule oxydée ;
- un amidon traité aux enzymes ;
- un phosphate de monoamidon ;
- un glycérolamidon ;
- un phosphate de diamidon estérifié au trimétaphosphate de sodium ;
- un phosphate de diamidon phosphaté ;
- un phosphate de diamidon acétylé ;
- un acétate d'amidon estérifié à l'anhydride acétique ;
- un acétate d'amidon estérifié à l'acétate de vinyle ;
- un adipate de diamidon acétylé ;
- un glycérol de diamidon acétylé ;
- un amidon hydroxypropylique ;
- un phosphate de diamidon hydroxypropylique ;
- un glycérol de diamidon hydroxypropylique ;et
- un octényle succinate d'amidon sodique.

L'agent stabilisant **D** est présent à raison de 30 à 49.5 % en poids par rapport au poids total de ladite partie **A.**

Selon un autre mode de réalisation préféré, l'agent stabilisant **D** est présent à raison de 30 à 45 % en poids par rapport au poids total de ladite partie **A.**

De manière particulièrement avantageuse, l'agent stabilisant **D** est un amidon de maïs.

De préférence, le composé **V** est un polyorganosiloxane présentant, par molécule, au moins deux groupes alcényle liés au silicium et comprenant :

(a) au moins deux motifs siloxyles de formule :

$$T_a\, Z_b\, SiO_{\frac{4-(a+b)}{2}} \quad (1.1)$$

dans laquelle :

- les symboles T sont des groupes alcényles en $C_2$-$C_6$ identiques ou différents,
- les symboles Z sont des groupes hydrocarbonés monovalent, identiques ou différents, choisi parmi le groupe constitué par un alkyle ayant de 1 à 8 atomes de carbone inclus, éventuellement substitués par au moins un atome d'halogène, avantageusement, parmi les groupes méthyle, éthyle, propyle et 3,3,3-trifluoropropyle et un aryle, avantageusement, parmi les radicaux xylyle, tolyle et phényle,
- a est égal à 1 ou 2, b est égal à 0, 1 ou 2 et la somme a + b est égale à 1, 2 ou 3, et

(b) éventuellement au moins un motif siloxyle de formule :

$$Z_c SiO_{\frac{4-c}{2}} \quad (1.2)$$

dans laquelle :

- le symbole Z a la même signification que ci-dessus et c est égal à 0, 1, 2 ou 3.

De façon avantageuse, le composé V est un polyorganosiloxane POS dont la viscosité comprise entre 10 et 200 000 mPa.s.

Toutes les viscosités dont il est question ici correspondent à une grandeur de viscosité dynamique qui est

mesurée, de manière connue en soi, à 25°C.

Selon une variante particulièrement avantageuse, la composition **X** selon l'invention comprend au moins deux polyorganosiloxanes **V** présentant, par molécule, au moins deux groupes alcényle liés chacun à un atome de silicium, de préférence vinyle, et dont les viscosités dynamiques **x1** et **x2** à 25°C sont comprises dans les intervalles 10 à 1000 mPa.s et de 1000 à 150 000 mPa.s respectivement.

Le polyorganosiloxane **V** peut être uniquement formé de motifs siloxyle de formule (1.1) ou peut contenir, en outre, des motifs syloxyle de formule (1.2). De même, il peut présenter une structure linéaire, ramifiée, cyclique ou en réseau.

Z est généralement choisi parmi les radicaux méthyle, éthyle et phényle et le plus souvent 60 % molaire (ou en nombre) au moins des radicaux Z sont des radicaux méthyle.

Des exemples de motifs siloxyles de formule (1.1) sont le motif vinyldiméthylsiloxyle, le motif vinylphénylméthylsiloxyle, le motif vinylméthylsiloxyle et le motif vinylsiloxyle.

Des exemples de motifs siloxyles de formule (1.2) sont les motifs siloxyle $SiO_{4/2}$, diméthylsiloxyle, triméthylsiloxyle, méthylphénylsiloxyle, diphénylsiloxyle, méthylsiloxyle et phénylsiloxyle.

Des exemples de polyorganosiloxane **V** sont des composés linéaires et cycliques comme : les diméthylpolysiloxanes à extrémités diméthylvinylsilyles, les copolymères (méthylvinyl) (diméthyl)polysiloxanes à extrémités triméthylsilyles, les copolymères (méthylvinyl) (diméthyl)polysiloxanes à extrémités diméthylvinylsilyles ; les méthylvinylpolysiloxanes cycliques.

En ce qui concerne le catalyseur **C** qui est un composé d'un métal du groupe du platine, il est bien connu de l'homme de métier. On peut, en particulier, utiliser les complexes du platine et d'un produit organique décrit dans les brevets US-A-3 159 601, US-A-3 159 602, US-A-3 220 972 et les brevets européens EP-A-0 057 459, EP-A-0 188 978 et EP-A-0 190 530, les complexes du platine et d'organosiloxanes vinylés décrits dans les brevets US-A-3 419 593, US-A-3 715 334, US-A-3 377 432 et US-A-3 814 730. La quantité pondérale du catalyseur **C,** calculée en poids de platine-métal, est généralement comprise entre 2 et 400 ppm, de préférence entre 5 et 200 ppm basés sur le poids total des polyorganosiloxanes **V** et **H.**

En ce qui concerne le polyorganosiloxane **H,** il s'agit d'un polyorganosiloxane qui présente, par molécule, au moins deux atomes d'hydrogène liés au silicium, ces groupes Si-H étant situés dans la chaîne et/ou en bout de chaîne.

Selon une variante préférée, lorsque le polyorganosiloxane **V** contient 2 groupes alcényles par molécule, alors le polyorganosiloxane **H** sera choisi de manière à contenir au moins 3 atomes d'hydrogène liés au silicium par molécule. Inversement, lorsque le polyorganosiloxane **H** contient 2 atomes d'hydrogène liés au silicium par molécule, alors le polyorganosiloxane **V** sera choisi de manière à contenir au moins 3 groupes alcényles par molécule.

Le polyorganosiloxane **H** est plus précisément un polyorganosiloxane comprenant :

(i) des motifs siloxyles de formule :

$$H_d\, L_e\, SiO_{\frac{4-(d+e)}{2}} \qquad (2.1)$$

dans laquelle :

- L est un groupe hydrocarboné monovalent, exempt d'action défavorable sur l'activité du catalyseur et choisi, de préférence, parmi les groupes alkyles ayant de 1 à 8 atomes de carbone inclus, éventuellement substitués par au moins un atome d'halogène, avantageusement, parmi les groupes méthyle, éthyle, propyle et 3,3,3-trifluoropropyle et ainsi que parmi les groupes aryles et, avantageusement, parmi les radicaux xylyle, tolyle et phényle,
- d est égal à 1 ou 2, e est égal à 0, 1 ou 2, la somme « d + e » est égale à 0, 1, 2 ou 3,

(2i) et éventuellement des autres motifs siloxyles de formule moyenne :

$$L_g\, SiO_{\frac{4-g}{2}} \qquad (2.2)$$

dans laquelle :

- L a la même signification que ci-dessus et g est égal à 0, 1, 2 ou 3.

La viscosité dynamique de ce polyorganosiloxane **H** est au moins égale à 10 mPa.s et, de préférence, elle est comprise entre 20 et 1000 mPa.s.

Le polyorganosiloxane **H** peut être uniquement formé de motifs siloxyle de formule (2.1) ou comporter en plus des motifs siloxyle de formule (2.2).

Le polyorganosiloxane **H** peut présenter une structure linéaire, ramifiée, cyclique ou en réseau.

Le groupe L a la même signification que le groupe Z décrit ci-dessus.

Des exemples de motifs siloxyle de formule (2.1) sont :

$H(CH_3)_2SiO_{1/2}$, $HCH_3SiO_{2/2}$, $H(C_6H_5)SiO_{2/2}$

Les exemples de motifs de formule (2.2) sont les mêmes que ceux donnés plus haut pour les motifs de formule (1.2).

Des exemples de polyorganosiloxane **H** sont des composés linéaires et cycliques comme :

- les diméthylpolysiloxanes à extrémités hydrogénodiméthylsilyle,
- les copolymères (diméthyl)(hydrogénométhyl)polysiloxanes à extrémités triméthylsilyles,
- les copolymères (diméthyl)(hydrogénométhyl)polysiloxanes à extrémités hydrogénodiméthylsilyles,
- les hydrogénométhylpolysiloxanes à extrémités triméthylsilyles, et
- les hydrogénométhylpolysiloxanes cycliques.

Le rapport du nombre d'atomes d'hydrogène liés au silicium dans le polyorganosiloxane **H** sur le nombre total de groupes à insaturation alcényle du polyorganosiloxane **V** est compris entre 0,4 et 10, de préférence entre 1 et 5.

Selon un mode de réalisation particulier, la partie **A** et/ou la partie **B** comprend au moins un composé choisi parmi le groupe constitué par : une charge renforçante **Q1,** une charge de bourrage **Q2,** un retardateur ou un inhibiteur **I** des réactions de polyaddition, une gomme polyorganosiloxane **G** présentant, par molécule, au moins deux groupes alcényle, de préférence vinyle, liés au silicium et dont la viscosité est supérieure à 1 000 Pa.s à 25°C, un polydiméthylsiloxane **F** utilisé à titre de diluant, un agent de coloration **K,** un agent plastifiant **P** choisi parmi le groupe constitué par l'huile de vaseline et une paraffine, un agent mouillant **M,** une résine silicone **R** et un agent biocide **N.**

En ce qui concerne la charge renforçante **Q1,** la charge habituellement utilisée consiste en une charge siliceuse. A titre de charges siliceuses susceptibles d'être mises en œuvre, conviennent toutes les silices précipitées ou pyrogénées (ou silices de combustion) connues de l'homme de l'art. Bien entendu, on peut utiliser aussi des mélanges de différentes silices. On préfère les silices de précipitation et/ou les silices de combustion ayant une surface spécifique BET supérieure à 40 $m^2/g$ et, plus précisément comprise entre 50 et 300 $m^2/g$. A titre plus préférentiel, on utilise les silices de combustion ayant les caractéristiques de surface spécifique mentionnées ci-avant. A titre encore plus préférentiel, on utilise les silices de combustion ayant une surface spécifique BET comprise entre 100 et 300 $m^2/g$. Généralement, cette charge renforçante présente une dimension moyenne des particules inférieures à 0,1 $\mu m$.

Ces silices peuvent être incorporées telles quelles ou après avoir été traitées par des composés orgnaosiliciques habituellement utilisés pour cet usage. Parmi ces composés figurent les méthylpolysiloxanes tels que l'hexaméthyldisiloxane, l'octaméthyldisiloxane, l'octaméthylcyclotétrasiloxane, des méthylpolysilazanes tels que l'hexaméthyldisilazane, l'hexaméthylcyclotrisilazane, des chlorosilanes tels que le diméthylchlorosilane, le triméthylchlorosilane, le méthylvinyldichlorosilane, le diméthylvinylchlorosilane, des alcoxysilanes tels que le diméthyldiméthoxysilane, le diméthylvinyléthoxysilane, le triméthylméthoxysilane. Lors de ce traitement, les silices peuvent accroître leur poids de départ jusqu'à un taux de 20 % de préférence 18 % environ. A noter que la charge minérale siliceuse particulaire peut avantageusement être mise en œuvre sous forme de la suspension obtenue en traitant la charge par application de la méthode, conforme à l'enseignement des demandes de brevets WO-A-98/58997 et WO-A-00/00853, prévoyant un traitement en deux temps de la charge par un agent de compatibilisation (choisi par exemple : s'agissant du premier temps de traitement, parmi un silazane, un siloxane hydroxylé, une amine, un acide organique ; et s'agissant du second temps de traitement, parmi un silazane) en opérant en présence du constituant POS (1). Dans le cas où pareil traitement conduit à un pH basique, on peut ajouter dans la dispersion un neutralisant tel que par exemple un acide faible. Pareil traitement particulier de la charge est intéressant quand on a besoin de conserver une excellente fluidité pour le matériau silicone (à l'état non réticulé). Ces charges, lorsqu'elles sont présentes, sont ajoutées à raison de 2 à 30 %, de préférence de 3 à 20 % par rapport au poids total de la composition.

En ce qui concerne la charge de bourrage **Q2,** elles ont généralement un diamètre particulaire supérieur à 0,1 $\mu m$ et elles sont choisies de préférence parmi le quartz broyé, les zircones, les argiles calcinées, les terres de diatomées, le carbonate de calcium éventuellement traité en surface, les silicates d'aluminium et/ou de sodium, des alumines, de l'oxyde de titane et des mélanges de ces espèces. Sur le plan pondéral, les charges de bourrage **Q2,** quand on en utilise, sont présentes dans le matériau silicone à raison de 5 à 60 % en poids par rapport au poids total de la composition **X.**

Les inhibiteurs I des réactions de polyaddition sont des composés bien connus. On peut en particulier utiliser les amines organiques, les oximes organiques, des diesters de diacide carboxylique, les alcools acétyléniques, les cétones acétyléniques tel que l'éthynylcyclohexanol, les vinylméthylcyclopolysiloxanes (voir par exemple US-A-3 445

420 et US-A-3 989 667). Quand il est présent dans la composition, l'inhibiteur est utilisé à raison de 0,005 à 5 % en poids, de préférence de 0,01 à 3 parties en poids pour 100 parties en poids du polyorganosiloxanes **V.**

En ce qui concerne le polydiméthylsiloxane **F** utilisé à titre de diluant, sa viscosité dynamique à 25°C est généralement comprise entre 10 et 5000 mPa.s et, de préférence, entre 20 et 1000 mPa.s.

Selon un mode de réalisation préféré, l'agent mouillant **M** est un tensioactif. Comme exemple d'agent mouillant **M,** on peut citer ceux de la demande internationale WO-A-2002102326, en particulier les composés suivants :

- un ester obtenu par estérification d'un acide gras en $C_{13}$ (acide laurique) par un poly(oxyéthylène)glycol contenant environ 9 motifs oxyéthylène, ayant un HLB de 13,1, commercialisé sous la dénomination LINCOL PE 400 ML;
- un alcool aliphatique saturé en $C_{13}$ polyéthoxylé contenant environ 8 motifs oxyéthylène (OE), ayant un HLB de 12,8, commercialisé sous la dénomination RHODASURF ROX;
- un alcool aliphatique saturé en $C_8$ polyalkoxylé contenant un nombre de motifs oxyéthylène et oxypropylène (OP) tel que la masse moléculaire Mw de l'alcool polyalkoxylé soit égale à environ 1000 g/mole, commercialisé sous la dénomination TEGOPREN LP 111 (en abréviation TA3) ;
- un alcool aliphatique saturé en $C_{10}$ - $C_{12}$ polyalkoxylé contenant environ 4 motifs oxyéthylène et 3 motifs oxypropylène, ayant un HLB de 7, commercialisé sous la dénomination ANTAROX FM 33 (en abréviation TA4) ;
- un alcool aliphatique saturé en $C_{12}$ polyéthoxylé contenant environ 2 motifs oxyéthylène, ayant un HLB de 8,1, commercialisé sous la dénomination RHODASURF OT/2.

Comme autres agents mouillants **M,** on peut citer des agents tensioactifs non-ioniques, ioniques ou amphotères. Les agents utilisés seront choisis au besoin sous une forme qui les rend compatibles pour le contact avec la peau et les muqueuses, en particulier buccales : ils doivent être non toxiques, non allergisants et non irritants aux doses d'emploi.

Parmi les agents tensioactifs non ioniques, on peut citer notamment : les acides gras polyalkoxylés ; les alkylphénols polyalkoxylés ; les alcools gras polyalkoxylés ; les amides gras polyalkoxylés ou polyglycérolés ; les amines grasses polyalkoxylées ; les polymères résultant de la condensation de l'oxyde d'éthylène et/ou de l'oxyde de propylène avec l'éthylèneglycol et/ou le propylèneglycol ; les polymères résultant de la condensation de l'oxyde d'éthylène et/ou de l'oxyde de propylène avec l'éthylènediamine ; les hydrocarbures terpéniques polyalkoxylés ; les polydiorganosiloxanes comportant des motifs siloxyles porteurs d'enchaînements oxyde d'éthylène et/ou d'enchaînements oxyde de propylène ; les polydiorganosiloxanes comportant des motifs siloxyles porteurs d'enchaînements de type polyol ; les silanes ou les polysilanes polyalkoxylés ; les alkylglucosides ; les alkylpolyglucosides ; les sucroéthers ; les sucroesters ; les sucroglycérides ; les esters de sorbitan ; les composés éthoxylés de ces dérivés de sucres ; et des mélanges de ces agents tensioactifs.

Parmi les agents tensioactifs anioniques, on peut citer notamment : les alkylbenzènesulfonates, les alkylsulfates, les alkyléthersulfates, les alkylaryléthersulfates, les alkylsuccinates, les alkylcarboxylates, les dérivés alkylés d'hydrolysats de protéine, les phosphates esters d'alkyle et/ou d'alkyléther et/ou d'alkylaryléther, où le cation est en général un métal alcalin ou alcalino-terreux ; et des mélanges des agents tensioactifs précités.

Parmi les agents tensioactifs cationiques, on peut citer notamment : les halogénures de trialkylbenzylammonium ; les halogénures de tétraalkylammonium ; et des mélanges de ces agents tensioactifs.

Parmi les agents tensioactifs amphotères, on peut citer notamment : les alkylbétaïnes, les alkyldiméthylbétaïnes, les alkylamidopropylbétaïnes, les alkylamidopropyldiméthylbétaïnes, les alkyltriméthyl-sulfobétaïnes ; les dérivés d'imidazoline tels que les alkylamphoacétates, alkylamphodiacétates, alkylampho-propionates, alkylamphodipropionates ; les alkylsultaïnes, les alkylamidopropyl-hydroxysultaïnes ; les produits de condensation d'acides gras et d'hydrolysats de protéines ; les dérivés amphotères des alkylpolyamines ; les protéines et hydrolysats de protéines ; et des mélanges de ces agents tensioactifs.

Des exemples spécifiques d'agents tensioactifs non ioniques sont:

(a) les alcools aliphatiques en $C_8$-$C_{22}$ polyalkoxylés contenant de 2 à 25 motifs alkoxylés, comme par exemple des motifs oxyéthylène (OE) et/ou oxypropylène (OP) ;

(b) les polydiorganosiloxanes comportant des motifs siloxyles porteurs d'enchaînements oxyde d'éthylène et/ou d'enchaînements oxyde de propylène ; à titre d'exemples, on peut citer les agents tensioactifs de formules I, II et III qui sont décrits dans le brevet US-A-4.657.959 ; et

(c) des mélanges d'agents tensioactifs (a) entre eux, des mélanges d'agents tensioactifs (b) entre eux et des mélanges d'un ou plusieurs agent(s) de type (a) avec un ou plusieurs agent(s) de type (b).

Le (ou les) agent(s) tensioactif(s) sont ajoutés en quantité au plus égale à 10 % et, de préférence, au plus égale à 5 % par rapport au poids total de la composition **X.**

En ce qui concerne l'agent de coloration **K,** on peut utiliser des pigments colorés minéraux et/ou organiques connus dans le domaine.

En ce qui concerne l'agent biocide **N** qui peut être mis en œuvre dans la composition selon l'invention, il est à noter qu'il est, de préférence, choisi dans le groupe de précurseur de chlore actif à base de composés N-chlorés comprenant :

- la chloramine B (sodium-N-chlorobenzène sulfonamide),
- la chloroamine T (sodium N-chloro-p-toluène sulfonamide),
- la dichloroamine T (N,N-dichloro-p-toluène sulfonamide),
- la N-trichlorométhylmercapto-4-cyclohexène-1,2-dicarboxylamide,
- l'halazone (acide benzoïque p-n-dichlorosulfonamide),
- la N-chlorosuccinimide,
- la trichloromélamine,
- la chloroazodine

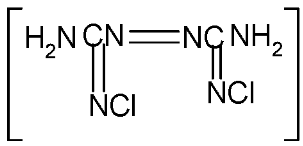

- les dérivés N-chloro des acides cyanuriques, de préférence l'acide trichloroisocyanurique et/ou le sodium dichloroisocyanurique dihydrate,
- les N-chlorohydantoïnes, de préférence la 1-bromo-3-chloro-5,5'-diméthylhydantoïne, ou la 1,3-dichloro-5,5'-diméthylhydantoïne,
- et leurs mélanges.

Ce groupe d'antiseptiques correspond sensiblement à la famille des N-chloramines qui comprend les dérivés des amines dans lesquelles une ou deux des valences de l'azote trivalent sont substituées par du chlore. En présence d'eau, les N-chloramines produisent de l'acide hypochloreux HClO ou des sels de cet acide tels que NaClO. HClO est NaClO sont des dérivés chlorés actifs, doués d'une grande capacité bactéricide, que l'on peut exploiter dans le cadre de l'invention (c'est en particulier le cas, lorsque ledit matériau est destiné à la prise d'empreintes dentaires en bouche). Avantageusement, peut être associé à au moins un auxiliaire antiseptique différent des antiseptiques fonctionnant par libération de chlore et de préférence choisi dans le groupe des formulations comportant un ou plusieurs ammoniums quaternaires (par exemple, le chlorure de benzalkonium) et éventuellement au moins un activateur séquestrant, de préférence sélectionné parmi les complexants d'ions métalliques (par exemple, l'EDTA ou Acide Ethylène Diamine Tétracétique).

La concentration en agent(s) biocide(s), quand on en utilise, est au plus égale à 1 %, de préférence au plus égale à 0,8 %, et plus préférentiellement encore comprise entre 0,001 et 0,5 % en poids par rapport à la masse totale de la composition selon l'invention.

Selon un mode de réalisation particulier, on obtient une composition **X** selon l'invention par mélange des parties **A** et **B** dans des ratios en poids compris entre 1:10 à 1:1 respectivement et de préférence par mélange en poids de 1 partie de **A** et de 5 parties de **B.**

Selon un autre mode de réalisation particulier, la composition **X** selon l'invention est caractérisée en ce que:

1) la partie (**A**) comprend:

- au moins deux polyorganosiloxanes **V** présentant, par molécule, au moins deux groupes alcényle liés chacun à un atome de silicium, de préférence vinyle, et dont les viscosités dynamiques **x1** et **x2** à 25°C sont comprises dans les intervalles 10 à 1000 mPa.s et de 1000 à 150 000 mPa.s respectivement.
- de 30 à 49,5 % en poids par rapport au poids total de ladite partie (**A)** d'au moins un agent stabilisant **D** choisi parmi le groupe des amidons,
- une quantité catalytiquement efficace d'au moins un catalyseur **C** qui est un composé d'un métal du groupe du platine,
- éventuellement au moins une gomme polyorganosiloxane **G** présentant, par molécule, au moins deux groupes alcényle, de préférence vinyle, liés au silicium et dont la viscosité est supérieure à 1 000 000 mPa.s à 25°C,
- éventuellement au moins une charge renforçante **Q1** et/ou une charge de bourrage **Q2,**
- éventuellement au moins un retardateur ou un inhibiteur **I** des réactions de polyaddition,
- éventuellement au moins un polydiméthylsiloxane **F** bloquée à chacune des extrémités de chaîne par un motif triméthylsilyle utilisé à titre de diluant,

- éventuellement au moins un agent de coloration **K,**
- éventuellement une paraffine **P,**
- éventuellement au moins un agent biocide **N,**
- éventuellement au moins un agent mouillant **M,** et
- éventuellement au moins une résine silicone **R,** et

2) la partie **B** ne contient pas de catalyseur **C** et comprend :

- au moins un composé **H** qui est un polyorganosiloxane présentant par molécule au moins trois atomes d'hydrogène liés au silicium,
- éventuellement au moins un composé **V** qui est un polyorganosiloxane présentant, par molécule, au moins deux groupes alcényle, de préférence vinyle, liés au silicium,
- éventuellement au moins un agent stabilisant **D** choisi parmi le groupe des amidons,
- éventuellement au moins une gomme polyorganosiloxane **G** présentant, par molécule, au moins deux groupes alcényle, de préférence vinyle, liés au silicium et dont la viscosité est supérieure à 1 000 000 mPa.s à 25°C,
- éventuellement au moins une charge renforçante **Q1** et/ou une charge de bourrage **Q2,**
- éventuellement au moins un retardateur ou un inhibiteur **I** des réactions de polyaddition
- éventuellement au moins un polydiméthylsiloxane **F** bloquée à chacune des extrémités de chaîne par un motif triméthylsilyle utilisé à titre de diluant,
- éventuellement au moins un agent de coloration **K,**
- éventuellement une paraffine **P,**
- éventuellement au moins un agent biocide **N,** et
- éventuellement au moins une résine silicone **R.**

Selon un mode de réalisation avantageux, la composition **X** selon l'invention est obtenue par mélange des parties **A** et **B** dans des ratios en poids compris entre 1:10 à 1:1 respectivement et de préférence par mélange en poids de 1 partie de **A** et de 5 parties de **B.**

Un autre objet de l'invention concerne un matériau **MA** ou un élastomère **E** obtenu par réticulation et/ou durcissement de la composition **X** selon l'invention et telle que définie ci-dessus.

Enfin, un dernier objet de l'invention concerne l'utilisation de la composition **X** selon l'invention et telle que définie ci-dessus ou du matériau **MA** ou élastomère **E** selon l'invention et tel que défini ci-dessus pour la prise d'empreinte dentaire, la fabrication des tampons employés dans les techniques de tampographie, la réalisation d'orthèse en podologie, ou la prise d'empreinte du conduit auditif.

Cette utilisation, dans une modalité de réalisation préférée, consiste à faire en sorte que la réticulation de l'élastomère silicone s'initie par mélange des parties **A** et **B,** à reproduire l'empreinte et à laisser se poursuivre la réticulation jusqu'à ce que l'élastomère ait suffisamment réticulé et soit suffisamment dur.

Selon un autre mode d'utilisation, le matériau **MA** ou élastomère **E** tel que décrit ci-dessus est destiné à la fabrication de tampons tels que ceux utilisés dans les techniques de tampographie, où il est intéressant de pouvoir disposer d'un matériau ayant de hautes propriétés mécaniques, dont on peut moduler l'énergie de surface par ajout d'agent(s) tensioactif(s) tout en conservant le niveau de fluidité qui est nécessaire à la fabrication des tampons par moulage. Cette autre utilisation, dans une modalité préférée de réalisation, consiste à faire en sorte que la réticulation de l'élastomère silicone s'initie par mélange des parties **A** et **B,** à conformer par moulage, de manière connue en soi, un objet ayant la forme du tampon souhaité et à laisser se poursuivre la réticulation jusqu'à ce que l'élastomère ait suffisamment réticulé et soit suffisamment dur.

Bien que la réticulation par des réactions de polyaddition entre les polyorganosiloxane **V** et **H** puisse être initiée et développée déjà à une température voisine de la température ambiante (23°C), on peut également réaliser la réticulation par voie thermique (en chauffant par exemple à une température allant de 60°C à 110°C) et/ou par rayonnement électromagnétique (rayonnement d'électrons accélérés ou "electron beam") et/ou par rayonnement infrarouge.

Les exemples qui suivent sont donner pour aider à la compréhension de la présente invention et ne doivent pas être interprétés comme en limitant l'étendue.

**EXEMPLE :**

a) Liste des matières premières utilisées :

- Polyorganosiloxane vinylé **V-1** : Huile polydiméthylsiloxane bloquée à chacune des extrémités des chaînes par un

motif siloxyle $(CH_3)_2(Vi)SiO_{1/2}$ de viscosité 100 000 mPa.s (avec Vi= groupement vinyle).

- Polyorganosiloxane vinylé **V-2:** Huile polydiméthylsiloxane bloquée à chacune des extrémités des chaînes par un motif siloxyle $(CH_3)_2(Vi)SiO_{1/2}$ de viscosité de 600 mPa.s.
- Polydiméthylsiloxane (PDMS) **F-1** bloquée à chacune des extrémités de chaîne par un motif siloxyle $(CH_3)_3SiO_{1/2}$ et ayant une viscosité de 50 mPa.s.
- Agent stabilisant **D-1** : amidon de maïs.
- Empatâge **1:** Mélange: Polyorganosiloxane vinylé **V-2** (huile polydiméthylsiloxane bloquée à chacune des extrémités des chaînes par un motif siloxyle $(CH_3)_2(Vi)SiO_{1/2}$ de viscosité de 600 mPa.s) + charge **Q-1** qui est une silice de combustion traitée en surface par du D4 (octaméthylcyclotétrasiloxane - mélange 85 :15 en poids respectivement.
- Agent mouillant **M-1:** ester obtenu par estérification d'un acide gras en $C_{13}$ (acide laurique) par un poly(oxyéthylène)glycol contenant environ 9 motifs OE, ayant un HLB de 13,1, commercialisé sous la dénomination LINCOL PE 400 ML® .
- Catalyseur **C-1** : Platine.
- Charge de bourrage **Q2-1** : Sicron SA 600 : quartz broyé de diamètre particulaire moyen de 10 mm.
- Charge de bourrage **Q2-2** : Carbonate de calcium Calofort-S.
- Charge de bourrage **Q2-3** : Zéolithe (Zeolite 4 A/S)
- Agent de coloration **K.**
- Polyorganosiloxane hydrogéné **H-1** : poly(diméthyl)(hydrogénométhyl)siloxane bloquée à chacune des extrémités des chaînes par un motif siloxyle $H(CH_3)_2SiO_{1/2}$ ayant une viscosité de 300 mPa.s.

Tableau 1 : Formulations des Parties **A** des bi-composants

| Composants (p/p) | Comparatif 1 | Comparatif 2 | Comparatif 3 | Comparatif 4 | Comparatif 5 | Exemple 1 | Exemple 2 | Exemple 3 |
|---|---|---|---|---|---|---|---|---|
| Polyorganosiloxane vinylé **V-1** | 19,0 | 19,0 | 19,0 | 19,0 | 19,0 | 13,8 | 19,0 | 19,0 |
| Polyorganosiloxane vinylé **V-2** | 16,0 | 16,0 | 16,0 | 16,0 | 16,0 | 7,0 | 16 | 16 |
| PDMS **F-1** | 15,5 | 15,5 | 15,5 | 15,5 | 15,5 | 15,0 | 15,5 | 15,5 |
| Agent stabilisant **D-1** | 22,5 | 22,5 | 22,5 | 0 | 22,5 | 34,0 | 49,5 | 49,5 |
| Charge de bourrage **Q2-2** | 27,0 | 27,0 | 22,0 | 0 | 27,0 | 0 | 0 | 0 |
| Charge de bourrage **Q2-1** | 0 | 0 | 0 | 39,5 | 0 | 5,0 | 0 | 0 |
| Charge de bourrage **Q2-3** | 0 | 0 | 5,0 | 10,0 | 0 | 0,0 | 0 | 0 |
| Empatâge **1** | 0 | 0 | 0 | 0 | 0 | 25,0 | 0 | 0 |
| Agent mouillant **M-1** | 0 | 0 | 0 | 0 | 0 | 0,2 | 0 | 0 |
| Catalyseur **C-1** (ppm) | 240 | 375 | 240 | 240 | 253 | 100 | 240 | 240 |
| p/p= parties en poids | | | | | | | | |

Tableau 2 : Formulation unique pour toute les Parties **B** des bi-composants.

| Composants (p/p) | Quantité Parties en poids |
|---|---|
| Polyorganosiloxane vinylé **V-1** | 22,75 |
| Polyorganosiloxane vinylé **V-2** | 4,80 |
| Agent stabilisant **D-1** | 37,80 |
| Empatâge **1** | 20,00 |
| Agent mouillant **M-1** | 0,15 |
| Polyorganosiloxane **H-1** | 4,00 |
| Charge de bourrage **Q2-1** | 9,99 |
| Agent de coloration **K** | 0,40 |

Pour chaque bi-composant testé, les parties **A** et **B** conditionnées sous forme de cartouche sont placés dans un four et subissent un vieillissement accéléré à 60°C. Après une durée de 7 jours et 25 jours, on évalue les propriétés des matériaux obtenus par réticulation à une température ambiante de 23°C d'une composition obtenue par mélange de 5 parties en poids de la partie **B** avec 1 partie en poids d'une partie **A.**

Le mélange peut être réalisé, soit à la main dans un bécher, soit à l'aide d'une machine doseuse de laboratoire. On évalue après 7 jours et 25 jours à 60°C:

- le temps de travail (W.T. en minutes et secondes) des compositions résultants des mélanges des parties A et B comme exposés ci-dessus et selon la norme ISO-4823,
- la mémoire élastique selon la norme ISO-4823, et
- la dureté Shore A (DSA) après 8 minutes de réticulation.

Après 25 jours à 60°C, on détermine la présence ou non de noircissement de la cartouche contenant le catalyseur (Partie A).

Les résultats sont consignés dans le Tableau 3.

Tableau 3 : Propriétés mesurés pour chaque matériau obtenu à partir des bi-composants

| Propriétés | Comparati f 1 | Comparati f 2 | Comparati f 3 | Comparati f 4 | Comparati f 5 | Exempl e 1 | Exempl e 2 | Exemple 3 |
|---|---|---|---|---|---|---|---|---|
| W.T<br>DSA à t=0 | 2'00"<br>17,5 | 1'45<br>25,2 | 1'55"<br>20 | 2'10"<br>17,0 | 2'05"<br>20,5 | 1'45"<br>20,5 | 1'42"<br>21 | 1'50"<br>20 |
| W.T<br>DSA après 7 jours à 60°C | 1'55"<br>15,6 | Non mesuré | 1'55"<br>19,0 | 2'50"<br>18,5 | 2'50"<br>13,5 | Non mesuré | 2'00"<br>18 | 2'05"<br>17 |
| W.T/DSA après 25 jours à 60°C | Inhibition totale, aucun matériau élastomère n'est obtenu | | | | | 2'10" 15 | 2'15" 15 | 2'30" 11 |
| Noircissement de la cartouche contenant le catalyseur (Partie A) après 25 jours à 60°C | OUI | OUI | OUI | OUI | OUI | NON | NON | NON |

Les tests Comparatifs 1, 2, 3 et 5 montrent que même lorsque l'agent stabilisant D est présent dans la partie A et à des teneurs relativement élevées (22,5 % en poids par rapport au poids total de la partie **A**), cela n'empêche pas un noircissement de la cartouche contenant le catalyseur (Partie A) après 25 jours de stockage à 60°C. Ce qui n'est plus le cas pour les exemples 1, 2 et 3 selon l'invention qui ne présentent plus ce problème de noircissement des parties A qui comprennent le catalyseur au platine. De plus, après un vieillissement de 25 jours à 60°C des parties A et B avant utilisation, on constate que les Exemples selon l'invention permettent d'obtenir un élastomère ayant un temps de travail acceptable pour une utilisation dans le domaine des prises d'empreinte dentaire.

## Revendications

**1.** - Composition **X** réticulable et/ou durcissable par des réactions de polyaddition se présentant sous la forme d'un système bicomposant **S** comprenant au moins deux parties distinctes **A** et **B** destinées à être mélangées pour former ladite composition **X** dans lequel :

- la partie **A** comprend:

  (a) de 30 à 49,5 % en poids par rapport au poids total de la partie **A** d'au moins un agent stabilisant **D** choisi parmi le groupe des amidons,
  (b) au moins un polyorganosiloxane **V** présentant, par molécule, au moins deux groupes alcényle, de préférence vinyle, liés chacun à un atome de silicium,
  (c) au moins un catalyseur **C** qui est un composé d'un métal du groupe du platine, et

- la partie **B** comprend au moins un polyorganosiloxane **H** présentant, par molécule, au moins deux atomes d'hydrogène liés à un atome de silicium identique ou différent,

**2.** - Composition **X** selon la revendication 1 dans laquelle l'agent stabilisant **D** est présent à raison de 30 à 45 % en poids par rapport au poids total de ladite partie **A.**

**3.** - Composition **X** selon l'une des revendications précédentes dans laquelle la partie **A** et/ou la partie **B** comprend au moins un composé choisi parmi le groupe constitué par : une charge renforçante **Q1,** une charge de bourrage **Q2,** un retardateur ou un inhibiteur **I** des réactions de polyaddition, une gomme polyorganosiloxane **G** présentant, par molécule, au moins deux groupes alcényle, de préférence vinyle, liés au silicium et dont la viscosité est supérieure à 1 000 Pa.s à 25°C, un polydiméthylsiloxane **F** utilisé à titre de diluant, un agent de coloration **K,** un agent plastifiant **P** choisi parmi le groupe constitué par l'huile de vaseline et une paraffine, un agent mouillant **M,** une résine silicone **R** et un agent biocide **N.**

**4.** - Composition **X** selon la revendication 1 ou 2 ou 3 dans laquelle l'agent stabilisant **D** est un amidon de maïs.

**5.** - Composition **X** selon la revendication 3 dans laquelle l'agent mouillant **M** est un tensioactif.

**6.** - Composition **X** selon la revendication 1 comprenant au moins deux polyorganosiloxane **V** présentant, par molécule, au moins deux groupes alcényle liés chacun à un atome de silicium, de préférence vinyle, et dont les viscosités dynamiques **x1** et **x2** à 25°C sont comprises dans les intervalles 10 à 1000 mPa.s et de 1000 à 150 000 mPa.s respectivement.

**7.** - Composition **X** selon la revendication 1 obtenue par mélange des parties **A** et **B** dans des ratios en poids compris entre 1:10 à 1:1 respectivement et de préférence par mélange en poids de 1 partie de **A** et de 5 parties de **B.**

**8.** - Composition **X** selon la revendication 1 ou 6 **caractérisé en ce que** le composé **V** est un polyorganosiloxane présentant, par molécule, au moins deux groupes alcényle liés au silicium et comprenant :

  (a) au moins deux motifs siloxyles de formule :

$$T_a Z_b SiO_{\frac{4-(a+b)}{2}} \quad (1.1)$$

dans laquelle :

- les symboles T sont des groupes alcényles en $C_2$-$C_6$ identiques ou différents,
- les symboles Z sont des groupes hydrocarbonés monovalent, identiques ou différents, choisi parmi le groupe constitué par un alkyle ayant de 1 à 8 atomes de carbone inclus, éventuellement substitués par au moins un atome d'halogène, et un aryle,
- a est égal à 1 ou 2, b est égal à 0, 1 ou 2 et la somme a + b est égale à 1, 2 ou 3, et

(b) éventuellement au moins un motif siloxyle de formule :

$$Z_cSiO_{\frac{4-c}{2}} \quad (1.2)$$

dans laquelle :

- le symbole Z a la même signification que ci-dessus et c est égal à 0, 1, 2 ou 3.

**9.** - Composition **X** selon la revendication 1 dans laquelle :

1) la partie (**A)** comprend:

- au moins deux polyorganosiloxane **V** présentant, par molécule, au moins deux groupes alcényle liés chacun à un atome de silicium, de préférence vinyle, et dont les viscosités dynamiques **x1** et **x2** à 25°C sont comprises dans les intervalles 10 à 1000 mPa.s et de 1000 à 150 000 mPa.s respectivement.
- de 30 à 49,5 % en poids par rapport au poids total de ladite partie (**A)** d'au moins un agent stabilisant **D** choisi parmi le groupe des amidons,
- une quantité catalytiquement efficace d'au moins un catalyseur **C** qui est un composé d'un métal du groupe du platine,
- éventuellement au moins une gomme polyorganosiloxane **G** présentant, par molécule, au moins deux groupes alcényle, de préférence vinyle, liés au silicium et dont la viscosité est supérieure à 1 000 000 mPa.s à 25°C,
- éventuellement au moins une charge renforçante **Q1** et/ou une charge de bourrage **Q2,**
- éventuellement au moins un retardateur ou un inhibiteur **I** des réactions de polyaddition,
- éventuellement au moins un polydiméthylsiloxane **F** bloquée à chacune des extrémités de chaîne par un motif triméthylsilyle utilisé à titre de diluant,
- éventuellement au moins un agent de coloration **K,**
- éventuellement une paraffine **P,**
- éventuellement au moins un agent biocide **N,**
- éventuellement au moins un agent mouillant **M,** et
- éventuellement au moins une résine silicone **R,** et

2) la partie **B** ne contient pas de catalyseur **C** et comprend :

- au moins un composé **H** qui est un polyorganosiloxane présentant par molécule au moins trois atomes d'hydrogène liés au silicium,
- éventuellement au moins un composé **V** qui est un polyorganosiloxane présentant, par molécule, au moins deux groupes alcényle, de préférence vinyle, liés au silicium,
- éventuellement au moins un agent stabilisant **D** choisi parmi le groupe des amidons,
- éventuellement au moins une gomme polyorganosiloxane **G** présentant, par molécule, au moins deux groupes alcényle, de préférence vinyle, liés au silicium et dont la viscosité est supérieure à 1 000 000 mPa.s à 25°C,
- éventuellement au moins une charge renforçante **Q1** et/ou une charge de bourrage **Q2,**
- éventuellement au moins un retardateur ou un inhibiteur **I** des réactions de polyaddition
- éventuellement au moins un polydiméthylsiloxane **F** bloquée à chacune des extrémités de chaîne par un motif triméthylsilyle utilisé à titre de diluant,
- éventuellement au moins un agent de coloration **K,**
- éventuellement une paraffine **P,**

- éventuellement au moins un agent biocide **N,et**
- éventuellement au moins une résine silicone **R.**

**10.** - Matériau **MA** ou élastomère **E** obtenu par réticulation et/ou durcissement de la composition **X** telle que définie selon l'une quelconque des revendications 1 à 9.

**11.** - Utilisation de la composition **X** telle que définie selon l'une quelconque des revendications 1 à 9 ou du matériau **MA** ou élastomère **E** tel que défini selon la revendication 10 pour la prise d'empreinte dentaire, la fabrication des tampons employés dans les techniques de tampographie, la réalisation d'orthèse en podologie, ou la prise d'empreinte du conduit auditif.

**Patentansprüche**

**1.** Zusammensetzung **X,** die durch Polyadditionsreaktionen vernetzbar und/oder härtbar ist und in Form eines Zweikomponentensystems **S** vorliegt, das mindestens zwei separate Teile **A** und **B** umfasst, die zum Mischen zur Bildung der Zusammensetzung **X** bestimmt sind, wobei:

- der Teil A Folgendes umfasst:

  (a) 30 bis 49,5 Gew.-%, bezogen auf das Gesamtgewicht des Teils **A,** mindestens eines Stabilisators **D,** der aus der Gruppe der Stärken ausgewählt ist,
  (b) mindestens ein Polyorganosiloxan **V,** das pro Molekül mindestens zwei Alkenylgruppen, vorzugsweise Vinylgruppen, die jeweils an ein Siliciumatom gebunden sind, aufweist,
  (c) mindestens einen Katalysator **C**, bei dem es sich um eine Verbindung eines Metalls der Platingruppe handelt, und

- der Teil **B** mindestens ein Polyorganosiloxan **H** umfasst, das pro Molekül mindestens zwei Wasserstoffatome, die an dasselbe Siliciumatom oder verschiedene Siliciumatome gebunden sind, aufweist.

**2.** Zusammensetzung **X** nach Anspruch 1, wobei der Stabilisator in einer Menge von 30 bis 45 Gew.-%, bezogen auf das Gesamtgewicht des Teils **A,** vorliegt.

**3.** Zusammensetzung **X** nach einem der vorhergehenden Ansprüche, wobei der Teil **A** und/oder der Teil **B** mindestens eine Verbindung umfasst, die aus der Gruppe bestehend aus einem verstärkenden Füllstoff **Q1**, einem streckenden Füllstoff **Q2,** einem Verzögerer oder einem Inhibitor **I** von Polyadditionsreaktionen, einem Polyorganosiloxangummi **G,** das pro Molekül mindestens zwei siliciumgebundene Alkenylgruppen, vorzugsweise Vinylgruppen, aufweist und dessen Viskosität größer als 1000 Pa.s bei 25 °C ist, einem Polydimethylsiloxan **F,** das als Verdünnungsmittel verwendet wird, einem Färbemittel **K,** einem Weichmacher **P,** der aus der Gruppe bestehend aus Vaselineöl und einem Paraffin ausgewählt ist, einem Netzmittel **M,** einem Silikonharz **R** und einem Biozid **N** ausgewählt ist.

**4.** Zusammensetzung **X** nach Anspruch 1 oder 2 oder 3, wobei es sich bei dem Stabilisator **D** um eine Maisstärke handelt.

**5.** Zusammensetzung **X** nach Anspruch 3, wobei es sich bei dem Netzmittel **M** um ein Tensid handelt.

**6.** Zusammensetzung **X** nach Anspruch 1, umfassend mindestens zwei Polyorganosiloxane **V,** die pro Molekül mindestens zwei Alkenylgruppen, die jeweils an ein Siliciumatom gebunden sind, vorzugsweise Vinylgruppen, aufweisen und deren dynamische Viskositäten **x1** und **x2** bei 25 °C in den Bereichen 10 bis 1000 mPa.s bzw. 1000 bis 150.000 mPa.s liegen.

**7.** Zusammensetzung **X** nach Anspruch 1, erhalten durch Mischen von Teilen A und B in jeweiligen Gewichtsverhältnissen zwischen 1:10 bis 1:1 und vorzugsweise durch Mischen von 1 Gewichtsteil **A** und 5 Gewichtsteilen **B.**

**8.** Zusammensetzung **X** nach Anspruch 1 oder 6, **dadurch gekennzeichnet, dass** es sich bei der Verbindung **V** um ein Polyorganosiloxan handelt, das pro Molekül zwei siliciumgebundene Alkenylgruppen aufweist und

(a) mindestens zwei Siloxyleinheiten der Formel:

$$T_a Z_b SiO_{\frac{4-(a+b)}{2}} \quad (1.1)$$

in der:

- die Symbole T für gleiche oder verschiedene $C_2$-$C_6$-Alkenylgruppen stehen,
- die Symbole Z für gleiche oder verschiedene einwertige Kohlenwasserstoffgruppen stehen, die aus der Gruppe bestehend aus einem Alkyl mit 1 bis 8 Kohlenstoffatomen inklusive und gegebenenfalls durch mindestens ein Halogenatom substituiert sind, und einem Aryl ausgewählt sind,
- a gleich 1 oder 2 ist, b gleich 0, 1 oder 2 ist und die Summe a + b gleich 1, 2 oder 3 ist, und

(b) gegebenenfalls mindestens eine Siloxyleinheit der Formel:

$$Z_c SiO_{\frac{4-c}{2}} \quad (1.2)$$

in der:

- das Symbol Z die gleiche Bedeutung wie oben hat und c gleich 0, 1, 2 oder 3 ist,

umfasst.

**9.** Zusammensetzung **X** nach Anspruch 1, wobei:

1) der Teil (**A**)

- mindestens zwei Polyorganosiloxane **V,** die pro Molekül mindestens zwei Alkenylgruppen, die jeweils an ein Siliciumatom gebunden sind, vorzugsweise Vinylgruppen, aufweisen und deren dynamische Viskositäten **x1** und **x2** bei 25 °C in den Bereichen 10 bis 1000 mPa.s bzw. 1000 bis 150.000 mPa.s liegen,
- 30 bis 49,5 Gew.-%, bezogen auf das Gesamtgewicht des Teils (**A**), mindestens eines Stabilisators **D,** der aus der Gruppe der Stärken ausgewählt ist,
- eine katalytisch wirksame Menge mindestens eines Katalysators **C**, bei dem es sich um eine Verbindung eines Metalls der Platingruppe handelt,
- gegebenenfalls mindestens ein Polyorganosiloxangummi **G,** das pro Molekül mindestens zwei siliciumgebundene Alkenylgruppen, vorzugsweise Vinylgruppen, aufweist und dessen Viskosität größer als 1.000.000 Pa.s bei 25 °C ist,
- gegebenenfalls mindestens einen verstärkenden Füllstoff **Q1** und/oder einen streckenden Füllstoff **Q2,**
- gegebenenfalls mindestens eine Verzögerer oder einen Inhibitor **I** von Polyadditionsreaktionen,
- gegebenenfalls mindestens ein Polydimethylsiloxan **F,** das an jedem der Kettenenden mit einer Trimethylsilyleinheit blockiert ist und als Verdünnungsmittel verwendet wird,
- gegebenenfalls mindestens ein Färbemittel K,
- gegebenenfalls mindestens ein Paraffin P,
- gegebenenfalls mindestens ein Biozid N,
- gegebenenfalls mindestens ein Netzmittel **M** und
- gegebenenfalls mindestens ein Silikonharz R umfasst und

2) der Teil **B** keinen Katalysator **C** enthält und

- mindestens eine Verbindung **H,** bei der es sich um ein Polyorganosiloxan handelt, das pro Molekül mindestens drei siliciumgebundene Wasserstoffatome aufweist,
- gegebenenfalls mindestens eine Verbindung **V,** bei der es sich um ein Polyorganosiloxan handelt, das pro Molekül mindestens zwei siliciumgebundene Alkenylgruppen, vorzugsweise Vinylgruppen, aufweist,
- gegebenenfalls mindestens einen Stabilisator **D,** der aus der Gruppe der Stärken ausgewählt ist,
- gegebenenfalls mindestens ein Polyorganosiloxangummi **G,** das pro Molekül mindestens zwei siliciumgebundene Alkenylgruppen, vorzugsweise Vinylgruppen, aufweist und dessen Viskosität größer als 1.000.000 Pa.s bei 25 °C ist,
- gegebenenfalls mindestens einen verstärkenden Füllstoff **Q1** und/oder einen streckenden Füllstoff **Q2,**
- gegebenenfalls mindestens eine Verzögerer oder einen Inhibitor **I** von Polyadditionsreaktionen,

- gegebenenfalls mindestens ein Polydimethylsiloxan **F,** das an jedem der Kettenenden mit einer Trimethylsilyleinheit blockiert ist und als Verdünnungsmittel verwendet wird,
- gegebenenfalls mindestens ein Färbemittel **K,**
- gegebenenfalls mindestens ein Paraffin **P,**
- gegebenenfalls mindestens ein Biozid **N** und
- gegebenenfalls mindestens ein Silikonharz **R** umfasst.

**10.** Material **MA** oder Elastomer **E,** erhalten durch Vernetzung und/oder Härtung der Zusammensetzung **X** gemäß einem der Ansprüche 1 bis 9.

**11.** Verwendung der Zusammensetzung **X** gemäß einem der Ansprüche 1 bis 9 oder des Materials **MA** oder Elastomers **E** gemäß Anspruch 10 zum Nehmen eines Zahnabdrucks, zur Herstellung von Tampons die bei Tampondrucktechniken eingesetzt werden, zur Herstellung einer Orthese in der Podologie oder zum Nehmen eines Abdrucks des Gehörgangs.

## Claims

**1.** Composition X crosslinkable and/or curable by polyaddition reactions, taking the form of a two-component system S which comprises at least two separate parts A and B intended for mixing to form said composition X, and in which:

- part A comprises:

(a) from 30% to 49.5% by weight, relative to the total weight of part A, of at least one stabilizer D selected from the group of starches,
(b) at least one polyorganosiloxane V having per molecule at least two alkenyl, preferably vinyl, groups which are each bonded to a silicon atom,
(c) at least one catalyst C which is a compound of a metal from the platinum group, and

- part B comprises at least one polyorganosiloxane H having per molecule at least two hydrogen atoms which are bonded to an identical or different silicon atom.

**2.** Composition X according to Claim 1, wherein the stabilizer D is present at from 30% to 45% by weight, relative to the total weight of said part A.

**3.** Composition X according to either of the preceding claims, wherein part A and/or part B comprises at least one compound selected from the group consisting of the following: a reinforcing filler Q1, a bulking filler Q2, a retarder or an inhibitor I of polyaddition reactions, a polyorganosiloxane gum G having per molecule at least two alkenyl, preferably vinyl, groups which are bonded to the silicon, and having a viscosity of greater than 1000 Pa.s at 25°C, a polydimethylsiloxane F used as diluent, a colorant K, a plasticizer P selected from the group consisting of liquid petroleum jelly and a paraffin, a wetting agent M, a silicone resin R, and a biocide N.

**4.** Composition X according to Claim 1 or 2 or 3, wherein the stabilizer D is a corn starch.

**5.** Composition X according to Claim 3, wherein the wetting agent M is a surfactant.

**6.** Composition X according to Claim 1, comprising at least two polyorganosiloxanes V having per molecule at least two alkenyl groups each bonded to a silicon atom, preferably vinyl, and having dynamic viscosities x1 and x2 at 25°C in the ranges 10 to 1000 mPa.s and from 1000 to 150 000 mPa.s respectively.

**7.** Composition X according to Claim 1, obtained by mixing parts A and B in weight ratios of between 1:10 to 1:1 respectively, and preferably by mixing, by weight, 1 part of A and 5 parts of B.

**8.** Composition X according to Claim 1 or 6, **characterized in that** the compound V is a polyorganosiloxane which has per molecule at least two alkenyl groups bonded to the silicon, and which comprises:

(a) at least two siloxyl units of formula:

$$T_a Z_b SiO_{\frac{4-(a+b)}{2}} \quad (1.1)$$

in which:

- the symbols T are identical or different $C_2$-$C_6$ alkenyl groups,
- the symbols Z are identical or different monovalent hydrocarbon groups selected from the group consisting of an alkyl having 1 to 8 carbon atoms inclusive, optionally substituted by at least one halogen atom, and an aryl,
- a is 1 or 2, b is 0, 1, or 2, and the sum a + b is 1, 2, or 3, and

(b) optionally at least one siloxyl unit of formula:

$$Z_c SiO_{\frac{4-c}{2}} \quad (1.2)$$

in which:

- the symbol Z has the same meaning as above and c is 0, 1, 2, or 3.

**9.** Composition X according to Claim 1, in which:

(1) part (A) comprises:

- at least two polyorganosiloxanes V having per molecule at least two alkenyl groups each bonded to a silicon atom, preferably vinyl, and having dynamic viscosities x1 and x2 at 25°C in the ranges 10 to 1000 mPa.s and from 1000 to 150 000 mPa.s respectively,
- from 30% to 49.5% by weight, relative to the total weight of said part (A), of at least one stabilizer D selected from the group of starches,
- a catalytically effective amount of at least one catalyst C which is a compound of a metal from the platinum group,
- optionally at least one polyorganosiloxane gum G having per molecule at least two alkenyl, preferably vinyl, groups which are bonded to the silicon, and having a viscosity of greater than 1 000 000 mPa.s at 25°C,
- optionally at least one reinforcing filler Q1 and/or one bulking filler Q2,
- optionally at least one retarder or one inhibitor I of polyaddition reactions,
- optionally at least one polydimethylsiloxane F blocked at each of the chain ends by a trimethylsilyl unit and used as diluent,
- optionally at least one colorant K,
- optionally a paraffin P,
- optionally at least one biocide N,
- optionally at least one wetting agent M,
- optionally at least one silicone resin R, and

(2) part B contains no catalyst C and comprises:

- at least one compound H which is a polyorganosiloxane having per molecule at least three hydrogen atoms bonded to the silicon,
- optionally at least one compound V which is a polyorganosiloxane having per molecule at least two alkenyl, preferably vinyl, groups bonded to the silicon,
- optionally at least one stabilizer D selected from the group of starches,
- optionally at least one polyorganosiloxane gum G having per molecule at least two alkenyl, preferably vinyl, groups which are bonded to the silicon, and having a viscosity of greater than 1 000 000 mPa.s at 25°C,
- optionally at least one reinforcing filler Q1 and/or one bulking filler Q2,
- optionally at least one retarder or one inhibitor I of polyaddition reactions,
- optionally at least one polydimethylsiloxane F blocked at each of the chain ends by a trimethylsilyl unit and used as diluent,

- optionally at least one colorant K,
- optionally a paraffin P,
- optionally at least one biocide N, and
- optionally at least one silicone resin R.

**10.** Material MA or elastomer E obtained by crosslinking and/or curing the composition X as defined in any one of Claims 1 to 9.

**11.** Use of the composition X as defined in any one of Claims 1 to 9 or of the material MA or elastomer E as defined in Claim 10 for taking dental impressions, for manufacturing pads employed in pad printing techniques, for producing podiatric orthoses, or for taking an impression of the auditory canal.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- US 5047444 A
- US 5118559 A
- US 5182316 A
- DE 19801657 A1
- US 6376569 B1
- WO 9317654 A1
- US 2006089427 A1
- US 3159601 A
- US 3159602 A
- US 3220972 A
- EP 0057459 A
- EP 0188978 A
- EP 0190530 A
- US 3419593 A
- US 3715334 A
- US 3377432 A
- US 3814730 A
- WO 9858997 A
- WO 0000853 A
- US 3445420 A
- US 3989667 A
- WO 2002102326 A
- US 4657959 A